# FASCICULE DE BREVET EUROPEEN

(11) **EP 1 458 724 B1**
(45) Date de publication et mention de la délivrance du brevet: **15.08.2007**
(21) Numéro de dépôt: 02745535.1
(22) Date de dépôt: 14.06.2002
(51) Int. Cl.: C07D 491/04, C07D 493/10, C07D 311/96, C07D 307/91, C07D 317/72

(54) **SYNTHESE TOTALE DE LA GALANTHAMINE, DE SES ANALOGUES ET DE SES DERIVES**
VOLLSTÄNDIGE SYNTHESE DER GALANTHAMIN, DEREN ANALOGEN UND DERIVATEN
TOTAL SYNTHESIS OF GALANTHAMINE, ANALOGUES AND DERIVATIVES THEREOF

(30) Priorité: 15.06.2001 FR 0107859
(43) Date de publication de la demande: 22.09.2004
(73) Titulaire: CENTRE NATIONAL DE LA RECHERCHE SCIENTIFIQUE (CNRS), 75016 Paris (FR)
(72) Inventeur: THAL, Claude, F-92330 Sceaux (FR); GUILLOU, Catherine, 91190 Gif-sur-Yvette (FR); BEUNARD, Jean-Luc, F-91400 Orsay (FR); GRAS, Emmanuel, F- 17300 Rochefort (FR); POTIER, Pierre, F-75007 Paris (FR)
(74) Mandataire: Warcoin, Jacques
(86) Numéro de dépôt international: PCT/FR2002/002045
(87) Numéro de publication internationale: WO 2002/102803

(56) Documents cités:
- DATABASE CA [en ligne] CHEMICAL ABSTRACTS SERVICE, COLUMBUS, OHIO, US; DRAGOJLOVIC, VELJKO: "Preparation of cyclopentenones by benzeneseleninic anhydride oxidation of cyclopentanones" retrieved from STN Database accession no. 131:31739 XP002188031 & J. CHEM. RES., SYNOP. (1999), (4), 256-257, 1240-1246,
- D.H.R. BARTON ET AL.: JOURNAL OF THE CHEMICAL SOCIETY, PERKIN TRANSACTIONS 1, 1980, pages 2209-2212, XP002188027 LETCHWORTH GB
- D.H.R. BARTON ET AL.: JOURNAL OF THE CHEMICAL SOCIETY, PERKIN TRANSACTIONS 1, 1982, pages 1919-1922, XP002188028 LETCHWORTH GB
- D.H.R. BARTON ET AL.: JOURNAL OF THE CHEMICAL SOCIETY, PERKIN TRANSACTIONS 1, 1982, pages 1947-1952, XP002188030 LETCHWORTH GB
- T.G. BACK: JOURNAL OF THE CHEMICAL SOCIETY, CHEMICAL COMMUNICATIONS, 1978, pages 278-279, XP002188066 LETCHWORTH GB
- GRAS E ET AL: "A formal synthesis of (+/-) lycoramine via an intramolecular Heck reaction" TETRAHEDRON LETTERS, ELSEVIER SCIENCE PUBLISHERS, AMSTERDAM, NL, vol. 40, no. 52, 24 décembre 1999 (1999-12-24), pages 9243-9244, XP004183640 ISSN: 0040-4039 cité dans la demande

## Description

La présente invention concerne un procédé de synthèse de la galanthamine, de ses analogues et ses dérivés, ainsi que les intermédiaires de synthèse correspondants.

La (-)-galanthamine de formule (A) 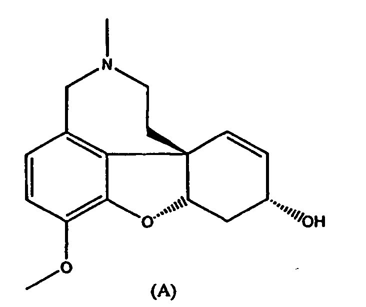 est un alcaloïde isolé de la famille des *Amaryllidaceae* qui agit comme un inhibiteur compétitif, sélectif et réversible de l'acétylcholinestérase (Harvey A.L. (1995), Pharmac. Ther, 68, 113).

Elle a été utilisée depuis plusieurs années dans le traitement de la myasthénie et dans certaines maladies neurologiques comme la poliomyélite, comme agent anticurare et comme agent parasymphaticomimétique.

Surtout, ce composé augmente les fonctions cognitives des malades atteints de la maladie d'Alzheimer, maladie caractérisée par des dommages de la neurotransmission cholinergique (Weinstock M. (1999), CNS Drugs, 12, 307). Actuellement, la galanthamine est commercialisée dans cette indication en Autriche et en Suède et devrait l'être prochainement dans les autres pays d'Europe et aux Etats-Unis.

La (-)-galanthamine peut être extraite de différentes sources végétales, notamment à partir de *Galanthus nivalis, G*. *narcissus, G. leucojum ou G. crinium,* mais en très faibles quantités insuffisantes pour un usage commercial.

La synthèse de la galanthamine a été réalisée pour la première fois par Barton D.H.R. et al. (J. Chem. Soc. (1962), 806), l'étape clé de ce procédé étant la cyclisation oxydative du phénol dont le rendement est seulement de 0,5%. De nombreuses équipes ont cherché à améliorer les rendements de ce procédé de synthèse pour permettre son utilisation à l'échelle industrielle; ainsi Czollner L. et al. (Tetrahedron Letters (1998), 39; 2087) ont obtenu un rendement de 45 à 50% et Krikorian et al. (Synthetic Communications (2000), 30 (16), 2833) des rendements de 60%. Toutefois, malgré l'augmentation de ces rendements, ces procédés restent difficiles à mettre en oeuvre à l'échelle industrielle.

Aussi, plusieurs équipes ont essayé de mettre au point des synthèses de la galanthamine, de ses analogues et dérivés par des voies non biomimétiques.

Ainsi, l'équipe de Ishisaki M. et al. (J..Org. Chem. (1993), 58, 3877) décrit une synthèse par réaction radicalaire de la lycoramine, alcaloïde de la famille de la galanthamine. Suite à une analyse rétrosynthétique de la lycoramine (1,2-dihydrogalanthamine), les auteurs montrent que le composé de structure (B) 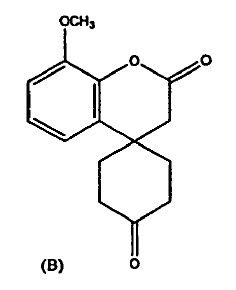 ne peut conduire qu'à la synthèse totale de la lycoramine, en 3 étapes avec un rendement global de 13%. Cet intermédiaire ne permet donc pas d'accéder à la galanthamine.

La galanthamine et ses dérivés comme la lycoramine se caractérisent par la présence d'un carbone quaternaire spirannique dont la création s'est révélée être l'étape limitante dans la synthèse totale.

Les inventeurs ont été les premiers à décrire une nouvelle stratégie pour former ce carbone quaternaire critique de la lycoramine (Gras E. et al., Tetrahedron Letters (1999), 40, 9243) en utilisant une réaction intramoléculaire de Heck; ils ont synthétisé l'intermédiaire de formule (C) 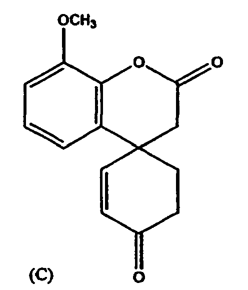 à partir duquel ils ont préparé la lycoramine par introduction d'un groupe amine, cyclisation de Pictet-Spengler, et réduction par LiAlH₄ dans le tétrahydrofurane selon la méthode décrite par Ishisaki M. (référence déjà citée).

Plus récemment, Trost B.M. et al. (J. Am. Chem. Soc. (2000), 122 (45), 11262) ont décrit la première synthèse énantiosélective complète de la (-)-galanthamine qui n'utilise pas le couplage oxydatif du phénol: ils réalisent une cyclisation par une réaction intramoléculaire de Heck en présence d'un ligand bidentate d'alkylphosphine et d'un catalyseur au palladium.

Toutefois, cette synthèse comprend 15 étapes et le rendement global est de 1%.

Pilger C. et al. (Synlett (2000), 8, 1163) et Parsons P.J. et al. (Tetrahedron Letters, (2001), 42, 2209) ont également décrit de nouvelles voies de synthèse du squelette de la 3-déoxygalanthamine en utilisant une réaction intramoléculaire sélective de Heck comme étape clé. Cette approche ne permet pas la création de la fonction allylique essentielle pour l'activité biologique.

Compte-tenu de l'importance du marché de la galanthamine, de ses dérivés et de ses analogues, il est absolument indispensable de mettre au point une synthèse qui soit facile, rapide et économiquement viable.

Or, les inventeurs ont trouvé de manière surprenante qu'en utilisant un agent oxydant mélangé à un support, ils obtenaient à partir du composé de formule (C) ou d'un analogue, la galanthamine ou ses dérivés dans des conditions compatibles avec une utilisation industrielle.

Aussi, l'invention a-t-elle pour objet un procédé de synthèse des composés de formule (1) 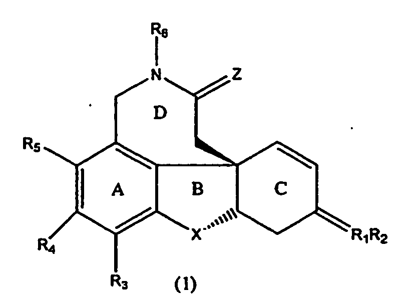 dans laquelle
soit R₁ représente un atome d'hydrogène et R₂ représente un groupe hydroxy, soit R₁ et R₂ forment ensemble =0,
R₃, R₄ et R₅ représentent chacun indépendamment l'un de l'autre un atome d'hydrogène, un groupe hydroxy ou un groupe (C₁-C₁₂) alcoxy,
R₆ représente un atome d'hydrogène, un groupe (C₁-C₁₂) alkyle, un groupe - (CH²) ₙNR₇R₈ ou un groupe -(CH₂)ₙN⁺R₇R₈R₉ où n = 1 à 12, R₇ et R₈ représentent chacun indépendamment l'un de l'autre, un atome d'hydrogène; un radical cyano; (C₁-C₄)alkyle; et R₉ représente un atome d'hydrogène, un radical cyano,
Z représente soit deux atomes d'hydrogène, soit un atome d'oxygène et
X représente soit un atome d'oxygène, soit un atome de soufre, soit un atome d'azote, soit un groupe -SO, soit un groupe -SO₂, soit un groupe -NR₆ où R₆ est tel que défini précédemment ou représente un groupe protecteur d'amine,
caractérisé en ce qu'il comprend une étape dans laquelle on réalise l'oxydation d'une cétone α,β-éthylénique de formule (10) 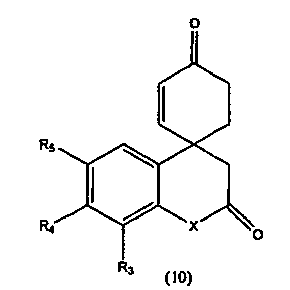 en spirodiénone de formule (11),

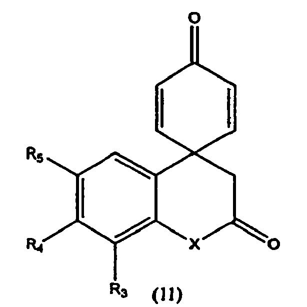

Dans un mode de réalisation avantageux du procédé, le support est un mélange de silice et d'alumine.

Dans un mode de réalisation particulièrement avantageux, le mélange de silice et d'alumine est un mélange 50/50.

Dans un mode préféré de réalisation, l'oxydation est réalisée en présence d'anhydride benzènesélininique mélangé à un support, de préférence inorganique. A titre d'exemple, on peut cité notamment les tamis moléculaires dont la taille est comprise de préférence entre 3 et 5 Å et les mélanges silice/alumine.

Les composés de formule (1) sont des analogues et des dérivés de la galanthamine, notamment les analogues basiques de la galanthamine dans lesquels l'atome d'azote du cycle D est salifiable et les analogues comportant une fonction iminium dans le cycle D, tels ceux décrits dans le demande internationale WO 97/03987 et dans l'article de Mary A. et al. (Bioorganic and Medicinal Chemistry (1998), 6, 1835). Ces composés possèdent 3 carbones asymétriques et peuvent donc exister sous la forme de stéréoisomères purs ou en mélanges. De préférence, le carbone 3 est de configuration α comme dans la galanthamine naturelle.

Dans un mode de réalisation avantageux du procédé selon l'invention, on fait réagir un dérivé de formule (6) 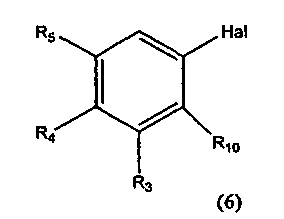 dans laquelle Hal représente un atome d'halogène choisi parmi les atomes de brome et d'iode, R₃, R₄ et R₅ sont tels que définis dans la revendication 1 et R₁₀ représente un groupe amine ou un groupe hydroxy, avec l'acide (1,4-dioxaspiro[4.5]déc-7-en-8-yl)-acétique de formule (7), 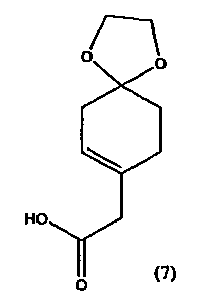 on obtient un composé de formule (8) 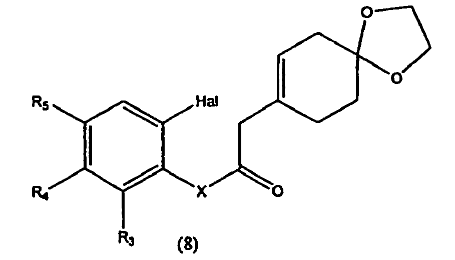 que l'on cyclise par une réaction intramoléculaire de Heck pour obtenir un composé de formule (9) 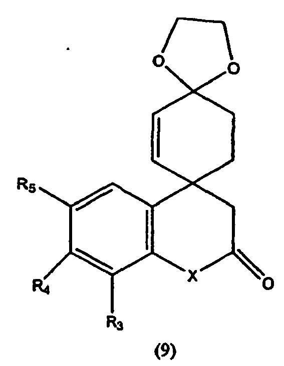 la réaction de cyclisation est réalisée dans des conditions classiques connues de l'homme du métier, notamment en présence d'un catalyseur au palladium ou d'un catalyseur précurseur de palladium 0, comme par exemple le tris (dibenzylidèneacétone)-dipalladium, et de dérivés de bidentate alkylphosphine comme par exemple du 1,2-bis (diphénylphosphino)éthane (dppe) ou du 1,2-bis(dicyclohexilphosphino)éthane (dcpe), et dans un mélange d'acétate de thallium et d'acétonitrile ou de diméthylacétamide; ensuite on déprotège la fonction dioxolane du composé de formule (9), par exemple en présence d'un accepteur d'hydrure comme par exemple le tétrafluoroborate de triphénylcarbénium ou l'hexafluorophosphate de triphénylcarbénium pour obtenir la cétone α,β-éthylénique de formule (10) 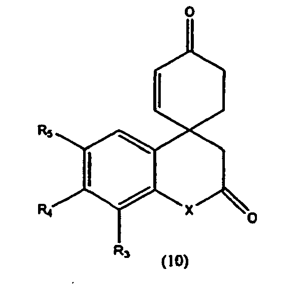 que l'on oxyde en présence d'anhydride benzèneseleninique additionné à un mélange de silice et d'alumine, de préférence 50/50, pour obtenir un composé de formule (11) 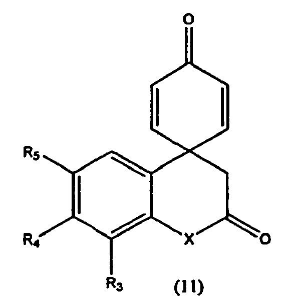 dans laquelle R_{3,} R₄ et R₅ et X sont tels que définis précédemment; sur ledit composé de formule (11) on introduit ensuite le groupe aminé par ouverture de la lactone avec une amine de formule NHR₆ où R₆ est tel que défini précédemment; cette réaction est accompagnée d'une réaction d'addition spontanée, de type Michael, du phénol généré intermédiairement, sur la diénone pour former avec un rendement quantitatif l'amide correspondant de formule (12) 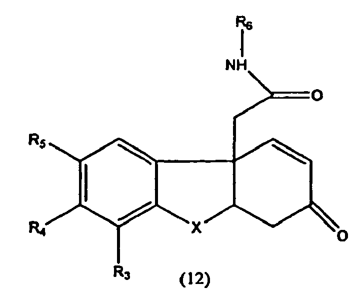 dans laquelle R₃, R₄, R₆ et X sont tels que définis précédemment .

Ensuite, on cyclise l'amide de formule (12), par exemple par une réaction de type Pictet-Spengler; ladite réaction peut être réalisée en présence de paraformaldéhyde et d'acide trifluoroacétique; on obtient alors un composé de formule (1a) 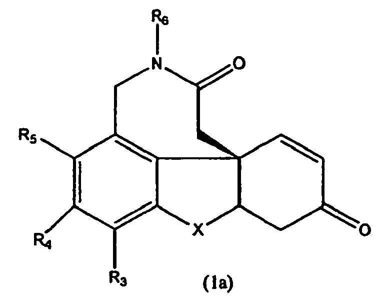 dont la réduction diastéréosélective, par exemple par le L-Sélectride^{®}, conduit au dérivé correspondant de formule (1b) 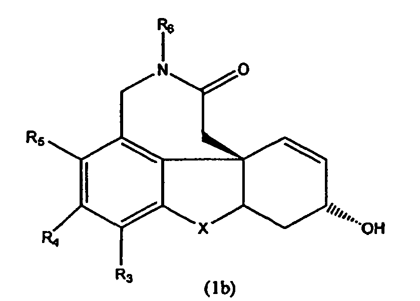 lui-même réduit dans des conditions classiques, pour obtenir le composé final correspondant de formule (1c)

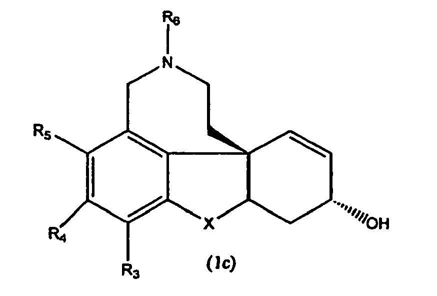

Selon un autre mode de réalisation de l'invention, dans le cas où l'on veut obtenir les composés de formule (1a) à (1c) optiquement actifs, alors on soumet le composé (12) ou (1a) à un dédoublement dans des conditions classiques connues de l'homme du métier et on procède selon les étapes décrites précédemment.

Dans un mode de réalisation particulièrement avantageux de l'invention, le procédé permet d'obtenir la galanthamine sous forme de racémate ou de ses isomères optiquement purs.

Le procédé selon l'invention permet d'obtenir la galanthamine, ses analogues et ses dérivés en un nombre d'étapes raisonnables compatibles avec une procédure industrielle.

L'invention a également pour objet des composés de formule (11) et (12) 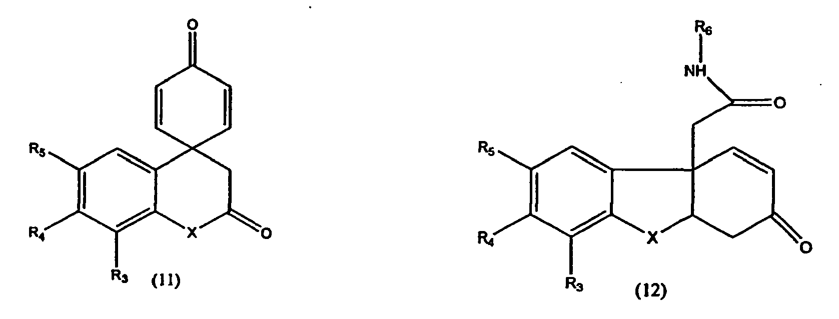 dans lesquelles R₃, R₄, R₅, R₆ et X sont tels que définis précédemment, utiles comme intermédiaires de synthèse.

L'invention a aussi pour objet des composés de formule (8) et (9) 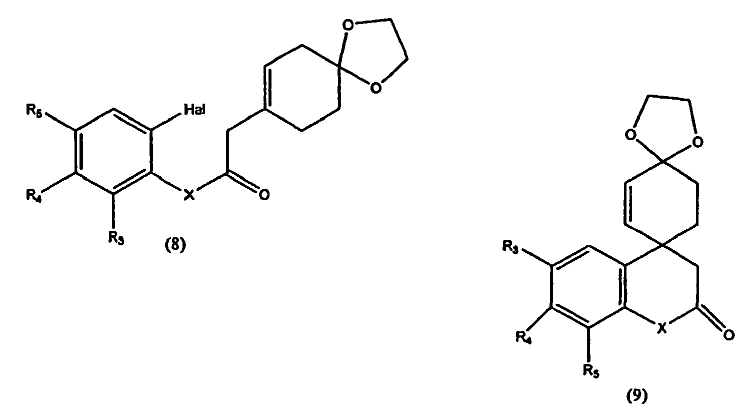 dans lesquelles Hal, R₃, R₄ et R₅ sont tels que définis précédemment et X représente un atome de soufre, un atome d'azote, un groupe -SO, un groupe -SO₂, un groupe -NR₆ où R₆ est tel que défini précédemment ou représente un groupe protecteur d'amine.

Les exemples qui suivent illustrent l'invention.

### Exemple 1: Synthèse totale de la galanthamine 1.1 (1',4'-dioxa-spiro[4.5]déc-7'-èn-8'-yl)-acétate de 2-iodo-6-méthoxy-phényle (8)

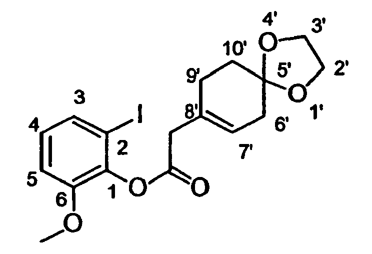

À 500 mg (2,52 mmol; 1,05 éq.) d'acide-(1,4-dioxaspiro[4.5]déc-7-èn-8-yl)-acétique (7) en solution dans 10 ml de CH₂Cl₂ à 0°C, sont additionnés progressivement 970 mg (5,05 mmol; 2,10 éq.) de EDC en solution dans 30 ml de CH₂Cl₂, puis 308 mg (2,52 mmol; 1,05 éq.) de déméthylaminopyridine en solution dans 10 ml de CH₂Cl₂. Le milieu réactionnel est agité pendant 15 minutes à 0°C, puis 600 mg (2,4 mmol; 1,00 éq.) de 2-iodo-6-méthoxy-phénol 6 en solution dans 10 ml de CH₂Cl₂ sont ajoutés. Après 5 heures d'agitation à température ambiante, le milieu réactionnel est hydrolysé et extrait avec de l'acétate d'éthyle (AcOEt). La phase organique est lavée avec une solution aqueuse saturée en chlorure de sodium, séchée sur sulfate de magnésium et évaporée sous vide. Le résidu est purifié par flash-chromatographie sur gel de silice (élution : Heptane / AcOEt: 7/3) pour conduire à 825 mg (80%) de (1',4'-dioxaspiro[4.5]déc-7'-èn-8'-yl)-acétate de 2-iodo-6-méthoxy-phényle 8 sous forme d'une huile incolore.
Analyse élémentaire: calculée pour C₁₇H₁₉IO₅: C, 47,46; H, 4,45; O, 18,59; mesurée: C, 47,34; H, 4,45; O, 18,41
I.R. (CHC1₃) v (cm⁻¹): 1764 (C=O); 1586 (C=C); 1468 (Car-C) ; 1267 (Car-O); 1117 (C-O)
S.M. (I.E., m/z): 430 (M⁺.); 250 (M⁺.-C₁₀H₁₂O₃); 180 (M⁺.-C₇H₇IO₂)
RMN ¹H (CDCl₃, 200 MHz) δ (ppm): 7,37 (1H, dd, J₄₋₃ = 6,0, J₄₋₅ = 3,0, H4) : 6,95-6,90 (2H, m, H3, H5) : 5,67 (1H, s large, H7'); 4,00 (4H, s, H2', H3'); 3,80 (3H, s, OCH₃); 3,32 (2H, s, CH2); 2,47-2,33 (4H, m, H6', H9'); 1,85 (2H, t, J = 6,4, H10')
RMN ¹³C (CDCl3, 75 MHz) δ (ppm): 168, 4 (C=O); 152,2 (C6); 141,7 (C1); 131,3 (C3); 130,1 (C8'); 128,2 (C5); 124,2 (C7'); 112,9 (C6) 107,4 (C5') ; 92,4 (C2) ; 64,8 (C2', C3') ; 56,2 (OCH3) ; 42,3 (CH2) ; 36,0 (C6'); 31,5 (C10') ; 28,4 (C9')

### 1.2 8-méthoxy-3,4,4a,8a-tétrahydro-1',4'-dioxa: chromèn-2-one-[4,8']-déc-6'-ène (9)

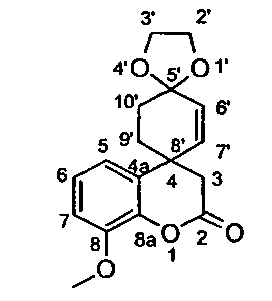

39 mg (0,04 mmol; 0,1 éq.) de tris(dibenzylidèneacétone)-dipalladium et 33 mg (0,08 mmol; 0,2 éq.) de 1,2-bis(diphénylphosphino)éthane en solution dans 10 ml de MeCN sont chauffés durant 30 minutes à 90°C. 117 mg (0,44 mmol; 1,2 éq.) d'acétate de thallium et 160 mg (0,37 mmol; 1,0 éq.) de (1', 4' -dioxaspiro[4.5] déc-7'-èn-8'-yl)-acétate de 2-iodo-6-méthoxy-phényle (8) sont alors additionnés au milieu réactionnel. Après 72 heures d'agitation à 90°C, le milieu réactionnel est filtré sur célite et évaporé sous pression réduite. La purification par chromatographie sur gel de silice (élution: Heptane/AcOEt: 7/3) du résidu obtenu conduit à 12,5 mg (67%) de 8-méthoxy-3,4,4a,8a-tétrahydro-1',4'-dioxaspiro-chromèn-2-one-[4,8']-déc-6'-ène 9 isolés sous la forme d'une poudre blanche.
P.F.: 101°C
Analyse élémentaire: calculée pour C₁₇H₁₈O₅: C, 67,54; H, 6,00; O, 26,46; mesurée: C, 67,37; H, 6,05; O, 26,46.
S.M.H.R. (I.C., m/z): calculée pour C₁₇H₁₉O₅⁺: 303,11543 ; mesurée: 303,12277
I.R. (CHCl₃) ν (cm⁻¹) : 1766 (C=O) ; 1583 (C=C) ; 1481 (CAR-C); 1233 (Car-O) ; 1090 (C-O)
S.M. (I.C., m/z) : 303 (MH⁺)
RMN ¹H (CDCl₃, 200 MHz) δ (ppm): 7,08 (1H, dd, J₆₋₅ = J₆₋₇ = 8,0, H6); 6,91 (1H, dd, J₇₋₆ = 8,0, J₇₋₅ = 1,5, H7); 6,81 (1H, dd, J₅₋₆ = 8,0, J₅₋₇ = 1,5, H5); 5,94 (1H, d, J_{6'-7'} = 10,0, H6'); 5,68 (1H, d, J7'-6' = 10,0, H7'); 4,05-3,97 (4H, m, H2', H3'); 3,90 (3H, s, OCH₃) ; 2,80 (1H, d, Jgem = 15,4, H3); 5,38 (1H, d, J_{gem} = 15,4, H3); 1,95-1,76 (4H, m, H9', H10')
RMN ¹³C (CDCl₃, 50 MHz) δ (ppm): 166, 7 (C2); 148,6 (C8); 140,1 (C8a); 134,8 (C6'); 131,4 (C7'); 128,6 (C4a); 124,1 (C5); 119,9 (C6) 112,4 (C7); 105,8 (C5'); 65,9 (C2', C3'); 60,1 (OCH3); 41,5 (C3); 39,0 (C4); 32,8 (C10'); 30,4 (C9')

### 1.3 8-méthoxy-3,4,4a,8a-tétrahydro-spiro-chromèn-2-one-[4,4']-cyclohex-2'-èn-l'-one (10)

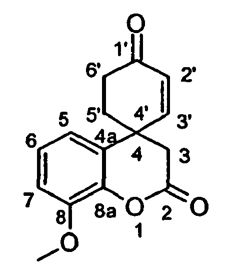

À 1,10 g (3,68 mmol; 1,0 éq.) de 8-méthoxy-3,4,4a,8a-tétrahydro-1',4'-dioxaspiro-chromèn-2-one-[4,8']-déc-6'-ène (9) solubilisés dans 50 ml de CH₂Cl₂ anhydre, sont ajoutés 1,21 g (3,68 mmol; 1,0 éq.) de tétrafluoroborate de triphénylcarbénium. Après 1 heure d'agitation à température ambiante, le milieu réactionnel est hydrolysé avec de l'eau puis extrait avec du CH₂Cl₂. La phase organique est lavée avec une solution aqueuse saturée en chlorure de sodium, séchée sur sulfate de magnésium et évaporée sous vide. La purification par flash-chromatographie sur gel de silice (élution: Heptane/AcOEt : 8/2 puis 5/5) du résidu obtenu conduit à 940 mg (100%) de 8-méthoxy-3,4,4a,8a-tétrahydro-spiro-chromèn-2-one-[4,4']-cyclohex-2'-èn-l'-one (10) isolés sous la forme d'une poudre blanche.
P.F.: 130°C
Analyse élémentaire: calculée pour C₁₅H₁₄O₄: C, 69,76 ; H, 5,46 ; O, 24,78 ; mesurée: C, 69,53 ; H, 5,61 ; O, 24,76.
I.R.(CHCl₃) ν (cm⁻¹) : 1770 (C=O); 1680 (O-C=O) ; 1585 (Car-C) ; 1480 (Car-C) ; 1248 (Car-O)
S.M. (I.C., m/z): 259 (MH⁺)
RMN ¹H (CDCl₃, 300 MHz) δ (ppm) : 7,13 (1H, dd, J₆₋₅= J₆₋₇ = 8,0, H6) : 6,98 (1H, dd, J₇₋₆ = 8,0, J₇₋₅ = 1,3, H7) ; 6, 75-(1H, dd, J₅₋₆ = 8,0, J₅₋₇ = 1,3, H5); 6,70 (1H, d, J_{3'-2'}= 10,1, H3'); 6,30 (1H, d, J2'-3' = 10, 1, H2') ; 3,92 (3H, s, OCH3); 2,93 (1H, d, J_{gem} = 15, 5, H3) ; 2,86 (1H, d, J_{gem} = 15,5, H3) ; 2,44 (2H, dd, J5'-6' = 6,0, J_{gem} = 12, H5') ; 2,19 (2H, dd, J6'-5' = 6,0, H6')
RMN ¹³C (CDCl₃, 75 MHz) δ (ppm): 198,7 (C1') ; 165,4 (C2) ; 150,1 (C3'); 148,9 (C8); 140,0 (C8a); 132,5 (C2'); 127,5 (C4a) ; 125,1 (C5) 118, 3 (C6) ; 112, 7 (C7) ; 56,3 (OCH3) ; 40,4 (C3) ; 39,2 (C4) ; 34,7 (C6') ; 33,5 (C5')

### 1.4 8-méthoxy-3,4,4a,8a-tétrahydro-spiro-chromèn-2-one-[4,4']-cyclohex-2',5'-dièn-1'-one (11)

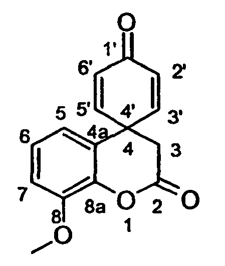

À 125 mg (0,48 mmol; 1,0 éq.) de 8-méthoxy-3,4,4a,8a-tétrahydro-spiro-chromèn-2-one-[4,4']-cyclohex-2'-èn-1'-one (10) en solution dans 12 ml de chlorobenzène anhydre contenant 400 mg de silice et 400 mg d'alumine, sont ajoutés 691 mg (1,92 mmol; 4,0 éq.) d'anhydride de benzènesélininique. Après 24 heures d'agitation au reflux, le milieu réactionnel est filtré sur fritté, rincé avec du MeOH et évaporé sous pression réduite. Le résidu est repris dans du CH₂Cl₂ puis lavé avec une solution aqueuse saturée en chlorure de sodium. La phase organique est séchée sur sulfate de magnésium et évaporée sous vide. La purification par flash-chromatographie sur gel de silice (élution: Heptane puis Heptane/AcOEt: 5/5) du résidu obtenu conduit à 62 mg (50%) de 8-méthoxy-3, 4,4a, 8a-tétrahydro-spiro-chromèn-2-one- [4, 4'] -cyclohex-2',5'-dièn-l'-one (11) isolés sous la forme d'une poudre jaune.
P.F.: 176-178°C
S.M.H.R. (I.C., m/z): calculée pour C₁₅H₁₃O₄⁺: 257,08139; mesurée: 257,08132
I.R. (CHCl₃) v (cm⁻¹): 1776 (C=O cétone) ; 1671 (C=O lactone) ; 1631 (C=C) ; 1281 (Car-O) ; 1179 (C-O)
S.M. (I.C., m/z): 257 (MH⁺); 238 (MH⁺-H₂O); 229 (MH⁺-CO)
RMN ¹H (CDCl₃, 300 MHz) δ (ppm) : 7,08 (1H, dd, J₆₋₅ = J₆₋₇= 8,1, H6) ; 6,96 (1H, d, J₇₋₆ = 8,1, H7) ; 6,89 (2H, d, J_{3'}-_{2'} = J_{5'-6'}= 10,3, H3', H5') ; 6,54 (1H, d, J₅₋₆= 8, 1, H5); 6,39 (2H, d, J_{2'-3'}= J_{6'-5'} = 10,3, H2', H6'); 3,90 (3H, s, OCH₃) ; 2,90 (2H, s, H3)
RMN ¹³C (CDCl₃, 75 MHz) δ (ppm): 184,5 (C1') : 164,6 (C2); 148,4 (C8) ; 148,1 (C3', C5') ; 140,8 (C8a) ; 130,0 (C2', C6') ; 125,4 (C6) ; 123,1 (C4a) ; 117,6 (C5) ; 113,0 (C7) ; 56,3 (OCH₃) ; 42,8 (C4) ; 38,5 (C3)

### 1.5 4a, 9b-Dihydro-6-méthoxy-9b-{[N-méthylamino)carbonyl] méthyl}-dibenzofuran-3-one (12)

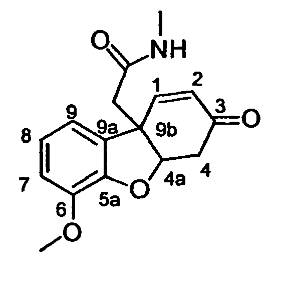

À 122 mg (0,43 mmol; 1,0 éq.) de 8-méthoxy-3,4,4a,8a-tétrahydro-spiro-chromèn-2-one-[4,4']-cyclohex-2',5'-dièn-1'-one (11) en solution dans 15 ml de tétrahydrofurane, sont ajoutés 0,13 ml (1,51 mmol; 3,5 éq.) de méthylamine en solution (40%) dans l'eau. Après 20 min d'agitation à température ambiante, le milieu réactionnel est lavé avec une solution aqueuse saturée en chlorure de sodium et extrait avec du CH₂Cl₂. La phase organique est lavée avec une solution aqueuse saturée en chlorure de sodium, séchée sur sulfate de magnésium et évaporée sous vide. La purification par flash-chromatographie sur gel de silice (élution: CH₂Cl₂/MeOH: 95/5) du résidu obtenu conduit à 137 mg (100%) de 4a, 9b-Dihydro-6-méthoxy-9b-{[N-méthylamino)carbonyl]méthyl}-dibenzofuran-3-one (12) isolés sous la forme d'une pâte jaune.
S.M.H.R. (I.C., m/z): calculée pour C₁₆H₁₈NO₄⁺: 288,12359 ; mesurée : 288,12350
I.R. (CHCl₃) ν (cm⁻¹): 3461 (N-H); 1680 (C=O cétone) (C=O amide) ; 1620 (C=C) ; 1282 (Car-O)
S.M. (I.E., m/z): 287 (M⁺.); 214 (M⁺. - C₃H₆NO)
RMN ¹H (CDCl₃, 300 MHz) δ (ppm) : 6,87 (3H, m, H7, H8, H9) ; 6,61 (1H, dd, J₁₋₄ₐ = 1, 8, J₁₋₂ = 10,0, H1) ; 6,05 (1H, s large, NH) ; 5,98 (1H, d, J₂₋₁ = 10,0, H2) ; 5,14: (1H, s large, H4a); 3,85 (3H, s, OCH₃) ; 3,16 (1H, dd, J₄-₄ₐ = 4,5, J_{gem} = 17,5, H4); 3,06 (1H, dd, J4-4a = 2, 5, J_{gem} = 17,5, H4) ; 2,91 (2H, d, J_{gem} = 15, 0, CH₂); 2,78 (3H, s, NCH₃) ; 2,76 (3H, s, NCH₃)
RMN ¹³C (CDCl₃, 75 MHz) δ (ppm) : 195,8 (C3) ; 169,4 (C=O); 147,3 (C1) ; 146,8 (C6) ; 145,0 (C9a) ; 131,8 (C5a) ; 127,0 (C2) ; 122,4 (Car) ; 115,0 (Car) ; 112, 5 (Car) ; 86,2 (C4a); 56,0 (OCH3) ; 47,7 (C9b) ; 43,4 (CH2) ; 38,7 (C4) ; 26,4 (NCH₃)

### 1.6 (±)-11-oxo-narwédine ou (±)-6-méthoxy-10-méthyl-galantham-1-ène-3,11 dione (la)

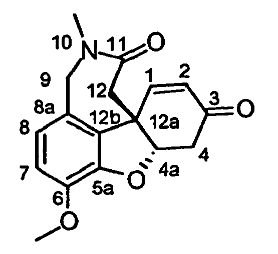

A 90 mg (0,31 mmol; 1,0 éq.) de 4a,9b-Dihydro-6-méthoxy-9b-{[N-méthylamino)carbonyl]méthyl}-dibenzofuran-3-one (12) en solution dans 10 ml de 1,2-dichloroéthane, sont ajoutés 38 mg (1,26 mmol; 4,0 éq.) de paraformaldéhyde et 0,3 ml (3,93 mmol; 12,5 éq.) d'acide trifluoroacétique. Après 20 heures d'agitation à 60°C, le milieu réactionnel est lavé avec une solution aqueuse saturée en hydrogénocarbonate de sodium et extrait avec du CH₂Cl₂. La phase organique est lavée avec une solution aqueuse saturée en chlorure de sodium, séchée sur sulfate de magnésium et évaporée sous vide. La purification par flash-chromatographie sur gel de silice (élution: CH₂Cl₂/MeOH: 95/5) du résidu obtenu conduit à 59 mg (63%) de (±)-11-oxo-narwedine (13) isolés sous la forme d'une pâte incolore.
S.M.H.R. (I.C. , m/z): calculée pour C₁₇H₁₇NO₄⁺ : 300,12359; mesurée : 300,12366
I.R. (CHCl₃) v (cm⁻¹) : 1723 (C=O cétone) ; 1680 (C=O amide) ; 1641 (C=C) ; 1509 (Car-C) ; 1285 (Car-O)
S.M. (I.C., m/z) : 300 (MH⁺)
RMN ¹H (CDCl₃, 300 MHz) δ (ppm) : 6,75 (2H, s, H7, H8) ; 6,39 (1H, dd, J₁₋₄ₐ = 2, 7, J₁₋₂ = 10,2, H1); 6,06 (1H, d, J₂₋₁ = 10,2, H2) ; 4,85 (1H, d, J₄ₐ₋₄ = 2, 7, H4a); 4,51 (1H, d, J_{gem} = 16,2, H9) ; 4,41 (1H, d, J_{gem} = 16,2, H9); 3,86 (3H, s, OCH₃); 3,17.(1H, dd, J₄₋₄ₐ = 2,7, J_{gem} = 17,7, H4) ; 3,06 (3H, s, NCH₃) ; 3,03 (1H, d, J_{gem} = 13,8, H12) ; 2,96 (1H, d, J_{gem} = 13,8, H12) ; 2,83 (1H, dd, J₄₋₄ₐ = 2,7,' J_{gem} = 17,7, H4)
RMN ¹³C (CDCl₃, 75 MHz) δ (ppm) : 193,7 (C3) ; 170,1 (C11). ; 147,6 (C6) ; 144,8 (C1, C5a) ; 129,8 (C12b) ; 127, 7 (C2) ; 124,9 (C8a) ; 120,3 (Car) ; 112,7 (Car) ; 87,1 (C4a) ; 56,3 (OCH₃) ; 51,9 (C9) ; 43,8 (C12a) ; 40,5 (C12) ; 36,4 (C4) ; 36,0 (NCH₃)

### 1.7 (±)-11-oxo-galanthamine ou (±)-6-méthoy-10-méthyl-galantham-1-èn-3α-ol-11-one (1b)

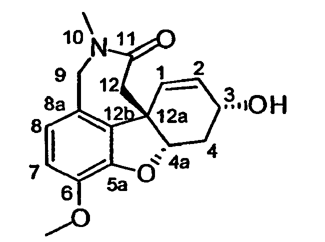

À 20 mg (0,07 mmol; 1,0 éq.) de (±)-11-oxo-narwedine 13 en solution dans 2 ml de THF, sont ajoutés 0,10 ml (0,10 mmol; 1,5 éq.) de L-sélectride^{®} en solution (1M) dans le THF. Après 1 heure d'agitation à -78°C, le milieu réactionnel est "quenché" avec du méthanol puis évaporé sous pression réduite. Le résidu est repris avec de l'AcOEt et lavé avec une solution aqueuse saturée en carbonate de sodium. La phase organique est lavée avec une solution aqueuse saturée en chlorure de sodium, séchée sur sulfate de magnésium et évaporée sous vide. La purification par plaque préparative (élution: CH₂Cl₂/MeOH: 95/5) du résidu obtenu conduit à 18,5 mg (93%) de (±)-10-oxo-galanthamine (14) isolés sous la forme d'une poudre blanche.
P.F. : 191-192°C
S.M.H.R. (I.C., m/z): calculée pour C₁₇H₂₀NO₄⁺: 302, 13924; mesurée : 302,13923
I.R.. (CHCl₃) v (cm⁻¹): 3557 (O-H) ; 1641 (C=O) (C=C); 1509 (Car-C) ; 1284 (Car-O)
S.M. (I.C., m/z): 302 (MH⁺) ; 284 (M⁺-OH); 258 (M⁺-C₂H₅N)
RMN ¹H (CDCl₃, 300 MHz) δ (ppm): 6,72 (2H, s, H7, H8) ; 6,04 (1H, dd, J₂₋₃= 5,4, J₂₋₁= 10,2, H2) ; 5,51 (1H, d, J₁₋₂ = 10, 2, H1) ; 4,76 (1H, t large, J₄ₐ₋₄= 1, 8, H4a) ; 4,46 (1H, d, J_{gem} = 15,9, H9); 4,34 (1H, d, J_{gem} = 15,9, H9); 4,17 (1H, t large, J₃₋₂ = 5, 4, H3) ; 3, 87 (3H, s, OCH₃) ; 3,04 (3H, s, NCH₃); 2, 81 (1H, d, J_{gem} = 14,1, H12) 2, 74 (1H, d, J_{gem} = 14, 1, H12) ; 2,68 (1H, dd, J₄₋₃ = 3,6, J_{gem} = 15, 9, H4); 2, 40 (1H, s large, OH); 2, 11 (1H, ddd, J₄₋₄ₐ=1
RMN ¹³C (CDCl₃, 75 MHz) δ (ppm): 171,0 (C11); 146,7 (C6); 144,9 (C5a); 132,2 (C12b); 128,4 (C1, C2); 125,2 (C8a); 120,2 (Car); 112,1 (Car); 112,1 (Car); 88,4 (C4a); 61,6 (C3); 56,2 (OCH₃); 52,1 (C9); 43,4 (C12a); 41,6 (C12); 36,0 (NCH₃) ; 29,3 (C4)

### 1.8 (±)-galanthamine (1c)

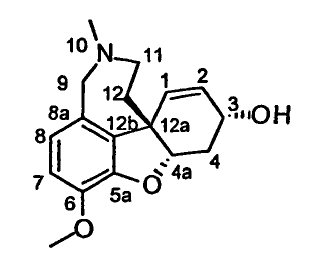

À 6 mg (0,15mmol; 5,5éq.) d'hydrure de lithium et d'aluminium en suspension dans 5 ml de DME, sont ajoutés goutte à goutte, à 0°C, 9 mg (0,06 mmol; 1,0 éq.) de (±)-11-oxo-galanthamine (1c) solubilisés dans 5 ml de DME. Après 12 heures d'agitation à 50°C, le milieu réactionnel est "quenché" par une solution de dithionite de sodium à 10% puis filtré sur célite (élution chloroforme). Le filtrat obtenu est lavé avec une solution aqueuse de dithionite de sodium à 10% et extrait avec du chloroforme. La phase organique est lavée avec une solution aqueuse saturée en chlorure de sodium, séchée sur sulfate de magnésium et évaporée sous vide. La purification par plaque préparative (élution: CH₂Cl₂/MeOH: 90/10) du résidu obtenu conduit à 7 mg (80%) de (±)-galanthamine (1c) isolés sous la forme d'une poudre blanche.
I.R. (CHCl3) v (cm⁻¹): 3562 (O-H) ; 1626 (C=C); 1599 (Car-C); 1508 (Car-C); 1280 (Car-O)
S.M. (I.C., m/z): 287 (M⁺.); 270 (M⁺.-OH); 244 (M⁺.-C₂H₅N) ; 230 (M⁺.-C₃H₇N); 216 (M⁺. -C₄H₉N)
RMN ¹H (CDCl₃, 250 MHz) δ (ppm): 6,67 (1H, d, J₁₋₂ = 8,3, Har) ; 6,63 (1H, d, J₁₋₂ = 8,3, Har); 6,07 (1H, dd, J₁₋₃ = 1, 0, J₁₋₂ = 10, 3, H1); 6,01 (1H, dd, J₂₋₃ = 4,3, J₂₋₁ = 10,3, H2); 4,62 (1H, s large, H4a); 4,14 (1H, t large, J = 5,0, H3); 4,11 (1H, d, J_{gem} = 15,3, H9) ; 3,84 (3H, S, OCH₃); 3,70 (1H, d, J_{gem} = 15,3, H9) ; 3,30 (1H, td, J₁₁₋₁₂ = 1,5, J_{gem} = 14,3; H11); 3,07 (1H, dt, J₁₁₋₁₂ = 3,5, J_{gem} = 14,3, H11); 2,69 (1H, dt, J₄₋₄ₐ = 2, 0, J_{gem} = 15, 8, H4); 2,48 (1H, s large, OH) ; 2,41 (3H, s, NCH₃) ; 2, 12 (1H, dd, J₁₂₋₁₁ = 3,5, J_{gem} = 13,5, H12); 2,01 (1H, ddd, J₄₋₄ₐ = 2, 0, J₄₋₃ = 5,0, J_{gem} = 15,8, H4); 1, 59 (1H, ddd, J₁₂₋₁₁ = 1,5, J₁₂₋₁₁ = 3,5, J_{gem} = 13,5, H9)
RMN ¹³C (CDCl₃, 75 MHz) δ (ppm) : 146,0 (C6) ; 144,3 (C5a) ; 133,2 (C12b) ; 129,2 (C8a) ; 127,8 (C1); 127,0 (C2) ; 122,3 (C8) ; 111, 4 (C7) ; 88,9 (C4a) ; 62,2 (C3) ; 60., 6 (C9) ; 56,1 (OCH₃) ; 53,9 (C11) ; 48,3 (C12a) ; 42,0 (NCH3) ; 33,8 (C12) ; 30,1 (C4)

### Exemple 2: Synthèse totale d'azagalanthamine

### 2.1 2-(1,4-dioxaspiro[4.5]dec-7-èn-8-yl)-N-(2'-iodo-6'-méthoxy-phényl)-acétamide (16)

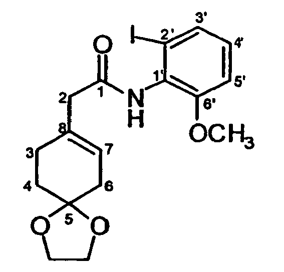

À une solution d'acide (1,4-dioxaspiro[4.5]dec-7-èn-8yl) acétique (567 mg ; 2,86 mmol; 1 éq) (7) et de 2-iodo-6-méthoxyaniline (1 g ; 2,86 mmol; 1 éq) dans du dichlorométhane anhydre (30 mL) sont ajoutés de l'iodure de 2-chloro-1-méthylpyridinium (1,46 g ; 5,73 mmol; 2-éq) et de la triéthylamine (3,98 mL ; 28,65 mmol; 10 éq). Le milieu réactionnel est porté au reflux pendant 20 heures. Après refroidissement et acidification avec une solution de HCl 1N jusqu'à pH = 5-6, le mélange est extrait avec du dichlorométhane. Les phases organiques sont lavées avec une solution aqueuse saturée en chlorure de sodium, séchées sur sulfate de magnésium et évaporées sous vide. La purification par flash chromatographie sur gel de silice (éluant: heptane/acétate d'éthyle 4/6) conduit à 1,10 g de 2-(1,4-dioxaspiro[4.5]dec-7-èn-8-yl)-N-(2'-iodo-6'-méthoxy-phényl)-acétamide (16) sous la forme d'une mousse jaune (rendement: 90 %).
Analyse élémentaire calculée pour C₁₇H₂₀INO₄ (P.M. : 429,25) C : 47, 57 ; H : 4,70 ; N : 3, 26 ; O : 14, 91 ; mesurée C : 47,39 ; H : 4,59 ; N : 3,01 ; O : 15,16.
I.R. (CHCl₃) ν (cm⁻¹) : 3382 (N-H) ; 1687 (C=O) .
S.M. (E.S.) m/z : 429,8 [M⁺H]⁺.
R.M.N. ¹H (CDCl₃; 300 MHz) δ (ppm) : 7,43 (dd, J=8,0, J=1,2; 1H; H3'); 7,18 (sl; 1H; NH); 6,98 (t, J=8,0; 1H; H4'); 6,91 (dd, J=8,0, J=1,2; 1H; H5'); 5,72 (sl; 1H; H4); 3,99 (s; 4H; Hdioxolane); 3,80 (s; 3H; OCH₃); 3,15 (s; 2H; H2); 2,45 (sl; 1H; H8); 2,37 (sl; 1H; H5); 2,37 (sl; 2H; H6); 1,85 (t, J=6,4; 2H; H7).
R.M.N. ¹³C (CDCl₃; 62,9 MHz) δ (ppm): 169,2 (C(O)NH); 155,5 (C6') ; 132,9 (C1') ; 130,8 (C3') ; 129,6 (C4'); 128,1 (C8) ; 124,9 (C7) ; 111, 7 (C5') ; 107,7 (C5) ; 99,8 (C2') ; 64,8 (Cdioxolane) ; 56,1 (OCH₃) ; 45,7 (C2); 35,9 (C6); 38,2 (C4); 35,0 (C3).

### 2.2 2-(1,4-dioxaspiro[4.5]dec-7-èn-8-yl)-N-(2'-iodo-6'-méthoxyphényN-méthyl-acétamide (17)

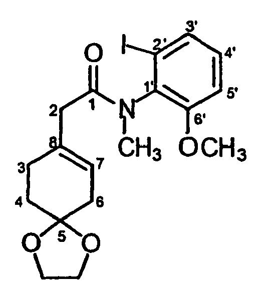

À une solution de 2-(1,4-dioxaspiro[4.5]dec-7-èn-8-yl)-N-(2'-iodo-6'-méthoxy-phényl)-acétamide (16) (1,88 g ; 4,39 mmol ; 1 éq.) dans 150 mL de tétrahydrofurane (THF) anhydre est ajoutée goutte à goutte à 0°C une suspension de NaH (263 mg ; 10,98 mmol; 2,5 éq.) dans le THF anhydre (120 mL). Après 15 minutes, le milieu réactionnel est laissé remonter à température ambiante et le sulfate de diméthyle (1,04 mL; 0,2 mmol; 2,5 éq.). est ajouté. Le milieu réactionnel est maintenu sous agitation à température ambiante pendant 2 heures puis la réaction est arrêtée par ajout d'une solution aqueuse d'hydrogénocarbonate de sodium saturée. Après extraction avec de l'éther, les phases organiques sont lavées par une solution aqueuse saturée de chlorure de sodium, séchées sur sulfate de magnésium et évaporées sous vide. La purification par flash chromatographie sur gel de silice (éluant: heptane/acétate d'éthyle 4/6) du résidu obtenu permet d'obtenir 26,9 mg du produit 2-(1,4-dioxaspiro[4.5]dec-7-èn-8-yl)-N-(2'-iodo-6'-méthoxy-phényl)-N-méthyl-acétamide (17) sous la forme d'une huile incolore (rendement: 86%). Le spectre RMN 1H révèle qu'il s'agit d'un mélange de deux rotamères en proportion 1:4 environ.
Analyse élémentaire calculée pour C₁₈H₂₂INO₄ (P.M.; 443,28) C: 48,77; H: 5,00; mesurée: C 48,98; H: 4,88.
I.R. (CHCl₃) ν (cm⁻¹) :1652 (C=O) ; 1602 (C=C).
S.M. (E.S.) m/z : 443,9 [M⁺H]⁺.
R.M.N. ¹H (CDCl₃ ; 300 MHz) δ (ppm) pour le rotamère majoritaire: 7,44 (dd, J=8,0, J=1,3 ; 1H ; H3') ; 7,01 (t, J=8,0 ; 1H ; H4') ; 6,90 (dd, J=8,0, J=1,3 ; 1H ; H5') ; 5,06 (sl ; 1H ; H7) ; 3,94 (s ; 4H ; Hdioxolane) ; 3,81 (s ; 3H ; OCH₃) ; 3,08 (s ; 3H ; NCH3) ; 2,73-2,65 (système AB, Jab=15,0 ; 2H ; H2a et H2b) ; 2,21 (sl ; 2H; H3) ; 2,19 (sl ; 2H ; H6) ; 1,78 (m ; 2H ; H4).
R.M.N. ¹³C (CDCl3; 75,4 MHz) δ (ppm) pour le rotamère majoritaire : 171,3 (C(O)NMe) ; 156,3 (C6'); 134,8 (C1') ; 131,1 (C8) ; 131,0 (C3') ; 130,7 (C4') ; 122,5 (C7) ; 111,7 (C5'); 107,9 (C5) ; 101,7 (C2'); 64,3 (Cdioxolane) ; 55,9 (OCH3) ; 42,2 (C2) ; 35,8 (C6) ; 34,5 (NCH₃) ; 31,0 (C4) ; 27,5 (C3).

### 2.3 8-méthoxy-1-méthyl-3,9',10'-dihydro-1H-1',9'-dioxaspiro-quinolin-2-one-[4.8']-déc-6'-ène (18)

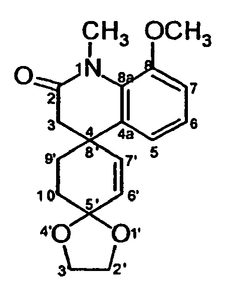

Une solution de tris(dibenzylidènacétone)dipalladium (36,8 mg ; 0,04 mmol; 0,05 éq.) et de 1,2-bis(diphénylphosphino)éthane (32,1 mg ; 0,08 mmol ; 0,1 éq.) dans le diméthylacétamide anhydre (10 mL) est maintenue sous agitation à température ambiante pendant 15 minutes. Une solution de 2-(1,4-dioxaspiro[4.5]dec-7-èn-8-yl)-N-(2'-iodo-6'-méthoxy-phényl)-N-méthyl-acétamide (17) (357 mg ; 0,81 mmol; 1 éq.) et de 1,2,2,6,6-pentaméthylpipéridine (582 µL; 3,22 mmol; 4 éq.) dans le diméthyl-acétamide anhydre (30 mL) est alors ajoutée goutte à goutte et le milieu réactionnel est porté à 110°C pendant 23 heures. Après refroidissement à température ambiante, ajout d'une solution aqueuse d'hydrogénocarbonate de sodium saturée et extraction avec de l'acétate d'éthyle, les phases organiques sont lavées par une solution aqueuse saturée de chlorure de sodium, séchées sur sulfate de magnésium et évaporées sous vide. Après purification par flash chromatographie sur gel de silice (éluant: heptane/acétate d'éthyle 4/6) 15,0 mg du produit 8-méthoxy-1-méthyl-3,9',10'-dihydro-1H-1',4'-dioxaspiro-quinolin-2-one-[4.8']-déc-6'-ène (18) sont obtenus sous la forme d'une huile jaune pâle (rendement: 80%).
S.M.H.R (I.C. , m/z): calculée pour C₁₈H₂₁NO₄⁺: 315,15; mesurée : 315,14794.
I.R. (CHC₁₃) ν (cm⁻¹) : 1659 (C=Olactame).
S.M. (E.S.) m/z : 316,2 [M⁺H]⁺ ; 338,2 [M⁺Na]⁺
R.M.N. ¹H (CDCl₃ ; 300 MHz) δ (ppm): 7,04 (t, J=8, 0 ; 1H ; H6) ; 6,88 (dd, J=8,0, J=1,3 ; 1H ; H5) ; 6,82 (dd, J=8,0, J=1, 3; 1H ; H7) ; 5,88 (d, J=10,0 ; 1H ; H6') 5,62 (d, J=10,0 ; 1H ; H7') ; 4,03-3,90 (m ; 4H ; Hdioxolane) ; 3,85 (s ; 3H ; OCH3) ; 3,39 (s ; 3H ; NCH₃) ; 2,58-2,48 (système AB, Jab=15,0 ; 2H ; H3a et H3b); 1,85-1,73 (m ; 4H ; H9' et H10').
R.M.N. ¹³C (CDCl₃; 75,4 MHz) δ (ppm): 170,1 (C(O)NMe); 150,4 (C8); 136,2 (C7'); 135,0 (C8a); 130,5 (C6'); 130,2 (C4a); 124,8 (C6); 119,4 (C5); 112,4 (C7); 105,3 (C5'); 64,8 et 64,6 (Cdioxolane); 56,2 (OCH₃) ; 44,0 (C3) ; 39, 2 (C4); 34,7 (NCH₃); 30,4 et 30,0 (C9' et C10').

### 2.4 8-méthoxy-1-méthyl-3,4-dihydro-1H-quinolin-2-one-[4.4']-cyclohex-5'-èn-1'-one (19)

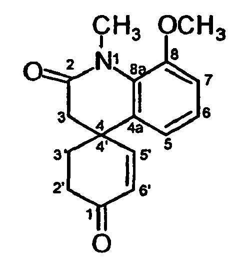

À 1,10 g (3,49 mmol; 1,0 éq.) de 8-méthoxy-1-méthyl-3,9',10'-dihydro-1H-l',4'-dioxaspiro-quinolin-2-one-[4.8']-déc-6'-ène (18) solubilisé dans 60 ml de CH₂CI₂ anhydre est ajouté 1,15 g (3,49 mmol; 1,0 éq.) de tétrafluoroborate de triphénylcarbénium. Après 1 heure d'agitation à température ambiante, le milieu réactionnel est hydrolysé avec de l'eau puis extrait avec du CH₂Cl₂. La phase organique est lavée par une solution aqueuse saturée de chlorure de sodium, séchée sur sulfate de magnésium et évaporée sous vide. La purification par flash-chromatographie sur gel de silice (élution: Heptane/AcOEt: 4/6) du résidu obtenu conduit à 898,5 mg de 8-méthoxy-1-méthyl-3,4-dihydro-1H-quinolin-2-one-[4.4']-cyclohex-5'-èn-l'-one (19) isolés sous forme d'une mousse jaune (rendement: 95 %).
S.M.H.R. (I.C., m/z) : calculée pour C₁₆H₁₇NO₃⁺: 271,12 ; mesurée : 271,12114.
I.R. (CHCl₃) v (cm⁻¹) : 3000 (C-H) ; 1677 (C=Oamide ; C=Oènone) ; 1370 (C=C) ; 1262 (C-OMe) ; 1086 (C-H.Ar).
S.M. (I.E.) m/z : 271 [M⁺· ] .
R.M.N. ¹H (CDCl₃ ; 250 MHz) δ (ppm) : 7,08 (q ; J 8;4 ; J = 7,6 ; 1H: H6) ; 6,96 (q ; J = 8,4 ; J = 1, 4 ; 1H ; H5) ; 6, 77 (q ; J = 7,6 ; J = 1,4 ; 1H ; H7) ; 6,63 (d ; J = 10,2 ; 1H ; H5') ; 3,88 (s ; 3H ; OCH3) ; 3,42 (s; 3H ; NCH3) ; 2,68 (système AB ; 2H, H3a et H3b ; Jab = 15,1) ; 2,43 (m ; 2H ; H3') ; 2,10 (t ; J = 6,9; 2H ; H2').
R.M.N. ¹³C (CDCl₃; 75,4 MHz) δ (ppm) : 198,8 (C1'); 169,0 (C2); 152, 4 (C5') ; 150,6 (C8); 132,6 (C8a); 131,1 (C6') ; 130,1 (C4a) ; 125,1 (C6) ; 118, 5 (C7) ; 112, 8 (C5) ; 56,1 (OCH₃) ; 43, 0 (C3) ; 39,8 (C4) ; 34,7 (NCH₃) ; 33,8 (C2') ; 31,5 (C3').

### 2.5 8-méthoxy-1-méthyl-3,4-dihydro-1H-quinolin-2-one-[4.4']-cyclohex-2',5'-dièn-1'-one (20)

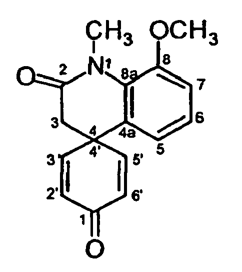

À 120 mg (0,44 mmol; 1,0 éq.) de 8-méthoxy-1-méthyl-3,4-dihydro-1H-quinolin-2-one-[4.4']-cyclohex-5'-èn-1'-one 19 en solution dans 10 ml de chlorobenzène contenant 400 mg d'alumine et 400 mg de silice, sont ajoutés 637 mg (1,77 mmol; 4,0 éq.) d'anhydride de benzènesélininique. Après 24 heures d'agitation au reflux, le milieu réactionnel est filtré sur fritté, lavé avec du MeOH et évaporé sous pression réduite. Le résidu est repris et extrait avec du CH₂C1₂, la phase aqueuse étant saturée en chlorure de sodium. La phase organique est lavée par une solution aqueuse saturée de chlorure de sodium, séchée sur sulfate de magnésium et évaporée sous vide. La purification par flash-chromatographie sur gel de silice (élution: Heptane/Acétate d'éthyle: 3,5/6,5) du résidu obtenu conduit à 73 mg de 8-méthoxy-1-méthyl-3,4-dihydro-1H-quinolin-2-one-[4.4']-cyclohex-2',5'-dièn-1'-one (20) isolés sous forme d'une mousse jaune (rendement: 61 %).
Analyse élémentaire calculée pour C16H15NO3 (P.M. : 269) C : 71,36 ; H : 5,61 ; N : 5,20 ; O : 17,82 ; mesurée: C: 69,33 ; H : 5,58 ; N : 5,19 ; O : 17,38.
I.R. (CHCl₃) ν (cm⁻¹) : 2962 (C-H); 1669 (C=O_{amide}; C=O_{diènone}); 1600 (C=C); 1370 (C-N); 1261 (C-OCH₃) ; 1097 (C-H.Ar).
S.M. (I.E.) m/z. 269 [M⁺·].
R.M.N. ¹H (CDCl₃ ; 300 MHz) δ (ppm) : 7,06 (q ; J = 7,9 ; J = 8,1 ; 1H ; H6) ; 6,95 (q ; J = 8,3 ; J =1,3 ; 1H ; H7) ; 6, 89 (d ; J = 9,8 ; 2H ; H3' et H5') : 6, 65 (q ; J = 1,0 ; J = 7,6 ; 1H ; H5) ; 6,37 (d ; J = 9,8 ; 2H ; H2' et H6') ; 3,88 (s ; 3H ; OCH₃) ; 3,47 (s ; 3H ; NCH₃) ; 2,70 (s ; 2H ; H3).
R.M.N. ¹³C (CDCl₃ ; 75,4 MHz) δ (ppm) : 185,0 (C1'); 168,2 (C2'); 150,4 (C8); 149,0 (C3' /C5') : 130,4 (C4a); 129,8 (C2'/C6'); 129,3 (C8a); 125,5 (C6); 118,2 (C5); 113,3 (C7); 56,0 (OCH₃); 43,5 (C3); 34,7 (NCH₃) .

### 2.6 (±)-11-oxo-azanarwédine (21)

Ce composé est préparé à partir de la 8-méthoxy-1-méthyl-3,4-dihydro-1H-quinolin-2-one-[4.4']-cyclohex-2',5'-dièn-1'-one (20) selon le mode opératoire décrit aux étapes 1.5 et 1.6 de l'exemple 1.

### 2.7 (±)azagalanthamine (22)

Ce composé est obtenu à partir de (21) selon le mode opératoire décrit aux étapes 1.7 et 1.8 de l'exemple.1.

## Revendications

1. Procédé de synthèse de composés de formule (1) 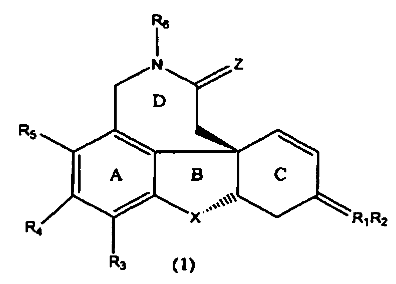 dans laquelle
soit R₁ représente un atome d'hydrogène et R₂ représente un groupe hydroxy, soit R₁ et R₂ forment ensemble =0,
R₃, R₄ et R₅ représentent chacun indépendamment l'un de l'autre un atome d'hydrogène, un groupe hydroxy ou un groupe (C₁-C₁₂) alcoxy,
R₆ représente un atome d'hydrogène, un groupe (C₁ -C₁₂) alkyle, un groupe -(CH₂)ₙNR₇R₈ ou un groupe - (CH₂) ₙN⁺R₇R_{B}R₉ où n = 1 à 12, R₇ et R₈ représentent chacun indépendamment l'un de l'autre, un atome d'hydrogène; un radical cyano; (C₁-C₄)alkyle; (C₁-C₄)alkyl-carbonyle et R₉ représente un atome d'hydrogène, un radical cyano, (C₁-C₄)alkyle,
Z représente soit deux atomes d'hydrogène, soit un atome d'oxygène et
X représente soit un atome d'oxygène, soit un atome de soufre, soit un atome d'azote, soit un groupe -SO, soit un groupe -SO₂ soit un groupe -NR₆ où R₆ est tel que défini précédemment ou représente un groupe protecteur d'amine,
**caractérisé en ce qu'**il comprend une étape dans laquelle on réalise l'oxydation d'une cétone α,β-éthylénique de formule (10) 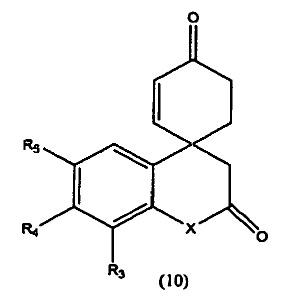 en spirodiénone de formule (11),

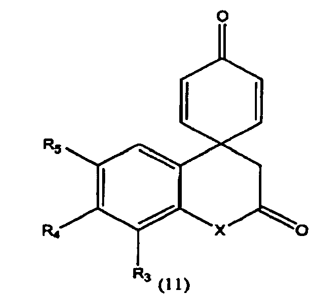

2. Procédé selon la revendication 1, **caractérisé en ce que** l'oxydation est réalisée en présence d'anhydride benzènesélininique mélangé à un support, de préférence inorganique.

3. Procédé selon l'une quelconques des revendications 1 et 2, **caractérisé en ce que** le support est choisi dans le groupe comprenant les tamis moléculaires et les mélanges de silice et d'alumine.

4. Procédé selon la revendication 3, **caractérisé en ce que** le mélange de silice et d'alumine est un mélange 50/50.

5. Procédé selon l'une quelconque des revendications 1 à 4, **caractérisé en ce qu'**on fait réagir un dérivé de formule (6) 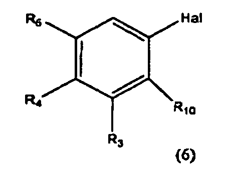 dans laquelle Hal représente un atome d'halogène choisi parmi les atomes de brome et d'iode, R₃, R₄ et R₅ sont tels que définis dans la revendication 1 et R₁₀ représente un groupe amine ou un groupe hydroxy, avec l'acide (1,4-dioxaspiro[4.5]déc-7-en-8-yl)-acétique de formule (7), 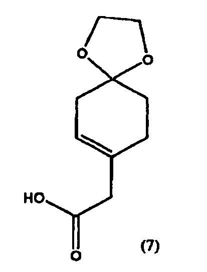 et on obtient un composé de formule (8) 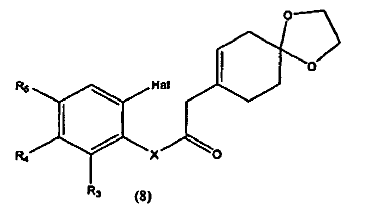 que l'on cyclise par une réaction intramoléculaire de Heck pour obtenir un composé de formule (9) 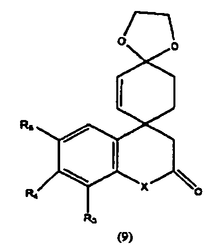 en présence d'un catalyseur au palladium ou précurseur de palladium 0 et de ligands bidentate d'alkylphosphine dans un solvant, puis on déprotège la fonction dioxolane du composé de formule (9) pour obtenir la cétone α,β-éthylénique de formule (10) 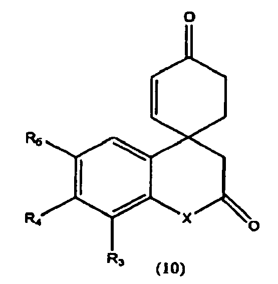 que l'on oxyde en présence d'anhydride benzèneselininique additionné à un mélange de silice et d'alumine 50/50 et on obtient un composé de formule (11) 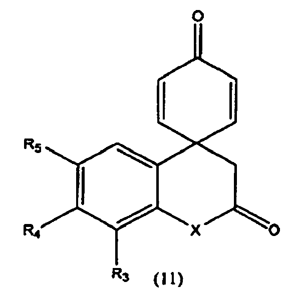 sur laquelle on fait réagir une amine de formule NHR₆ où R₆ est tel que défini dans la revendication 1 et on obtient, par ouverture de la lactone, l'amide correspondant de formule (12) 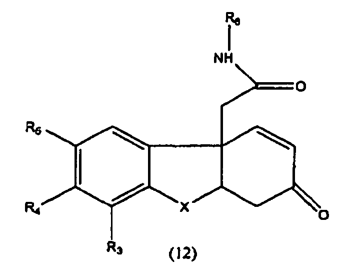 que l'on cyclise pour obtenir un composé de formule (1a) 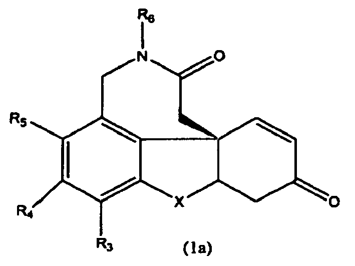 que l'on soumet éventuellement à une réduction diastéréosélective pour obtenir le dérivé de formule (1b) correspondant, 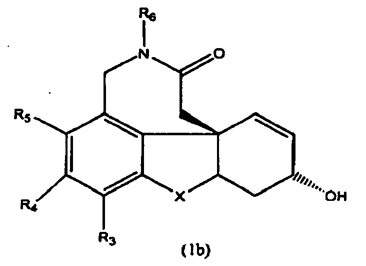 dont on peut également réduire la fonction amide, si on le souhaite, pour obtenir un composé de formule (1c).

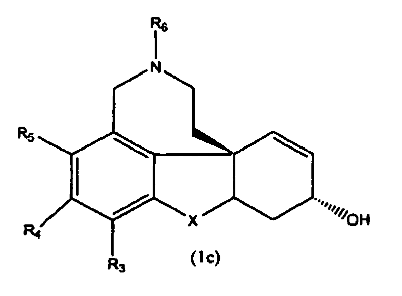

6. Procédé selon la revendication 5, **caractérisé en ce qu'**on réalise le dédoublement du composé de formule (12) puis on continue la synthèse et on obtient les composés de formule (1a) à (1c) sous leurs formes optiquement actives.

7. Procédé selon la revendication 5, **caractérisé en ce qu'**on réalise le dédoublement du composé de formule (1a) puis on continue la synthèse et on obtient les composés de formule (1b) à (1c) sous leurs formes optiquement actives.

8. Procédé selon l'une quelconque des revendications 1 à 7, **caractérisé en ce que** l'on prépare la galanthamine sous forme de racémate ou de ses isomères optiquement purs.

9. Composés de formules (11) et (12) 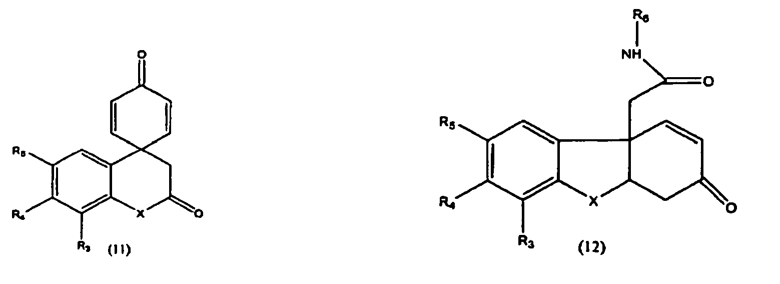 dans lesquelles R₃, R₄, R₅, R₆ et X sont tels que définis dans la revendication 1, utiles comme intermédiaires de synthèse dans un procédé selon l'une quelconque des revendications 1 à 7.

10. Composés selon la revendication 9, **caractérisés en ce que** R₃=OCH₃, R₄=R₅=H et X=O, NH ou N-CH₃.

11. Composés de formules (8) et (9) 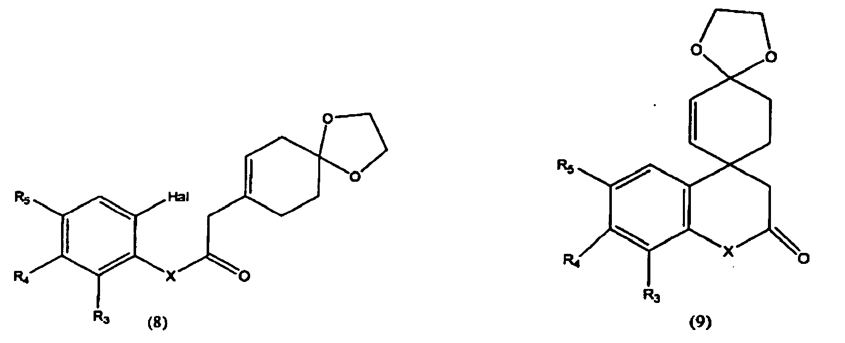 dans lesquelles Hal, R₃, R₄ et R₅ sont tels que définis dans la revendication 1 et X représente soit un atome de soufre, soit un atome d'azote, soit un groupe -SO, soit un groupe -SO₂, soit un groupe -NR₆ où R₆ est tel que défini dans la revendication 1 ou représente un groupe protecteur d'amine.

12. Composés selon la revendication 11, **caractérisés en ce que** Hal=I, R₃=OCH₃, R₄=R₅=H et X=NH ou N-CH₃.

13. Composés choisis parmi

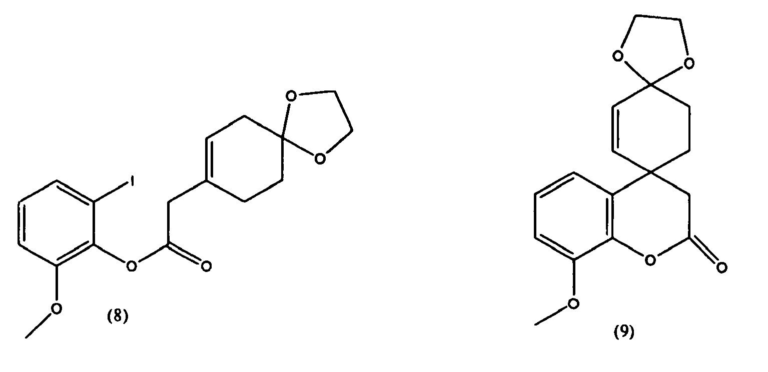

## Claims

1. Method of synthesizing compounds of formula (1) 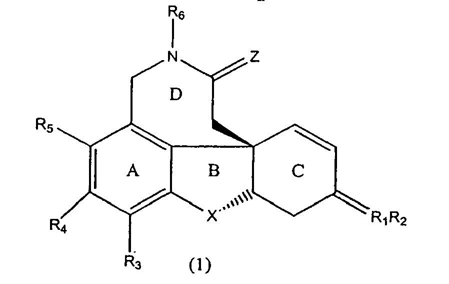 in which
either R₁ represents a hydrogen atom and R₂ represents a hydroxyl group, or R₁ and R₂ together form =O,
R₃, R₄ and R₅ represent each independently of one another a hydrogen atom, a hydroxyl group or a (C₁-C₁₂) alkoxy group,
R₆ represents a hydrogen atom, a (C₁-C₁₂)alkyl group, a group - (CH₂)ₙNR₇R₈ or a group - -(CH₂)ₙN⁺R₇R₈R₉ where n = 1 to 12, R₇ and R₈ represent each independently of one another a hydrogen atom; a cyano; (C₁-C₄) alkyl; or (C₁-C₄) alkyl-carbonyl radical and R₉ represents a hydrogen atom or a cyano or (C₁-C₄) alkyl radical,
Z represents either two hydrogen atoms or one oxygen atom, and
X represents alternatively an oxygen atom or a sulfur atom or a nitrogen atom or an -SO group or an -SO₂ group or a group -NR₆ where R₆ is as defined above or represents an amine-protective group,
**characterized in that** it comprises a step in which an α,β-ethylenic ketone of formula (10) 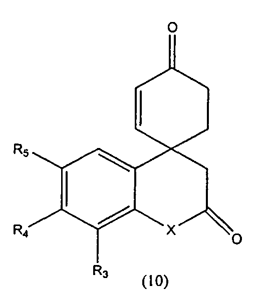 is oxidized to a spirodienone of formula (11),

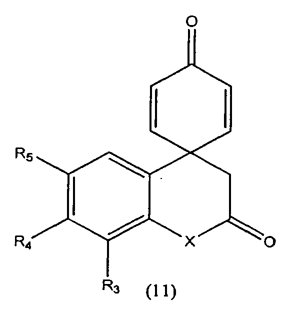

2. Method according to Claim 1, **characterized in that** the oxidation is performed in the presence of benzeneseleninic anhydride mixed with a support, preferably an inorganic support.

3. Method according to either of Claims 1 and 2, **characterized in that** the support is selected from the group consisting of molecular sieves and mixtures of silica and alumina.

4. Method according to Claim 3, **characterized in that** the mixture of silica and alumina is a 50/50 mixture.

5. Method according to any one of Claims 1 to 4,
**characterized in that** a derivative of formula (6) 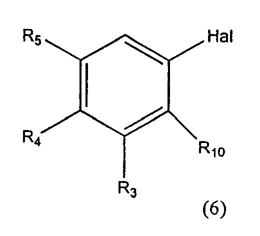 in which Hal represents a halogen atom selected from bromine and iodine atoms, R₃, R₄, and R₅ are as defined in Claim 1, and R₁₀ represents an amine group or a hydroxyl group is reacted with (1,4-dioxaspiro[4.5]dec-7-en-8-yl)acetic acid of formula (7), 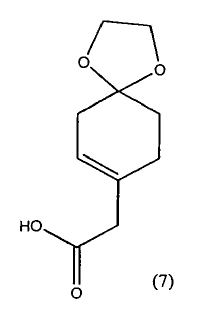 and a compound of formula (8) 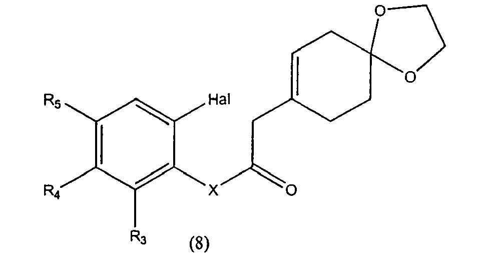 is obtained which is cyclized by an intramolecular Heck reaction to give a compound of formula (9) 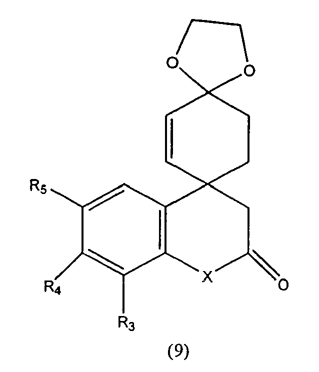 in the presence of a palladium or a palladium(0) precursor catalyst and of bidentate alkylphosphine ligands in a solvent, then the dioxolane function of the compound of formula (9) is deprotected to give the α,β-ethylenic ketone of formula (10) 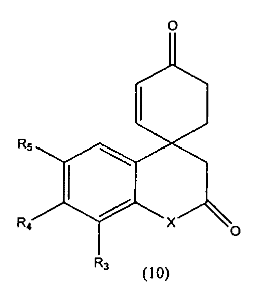 which is oxidized in the presence of benzeneseleninic anhydride, to which a mixture, preferably 50/50, of silica and alumina has been added, to give a compound of formula (11) 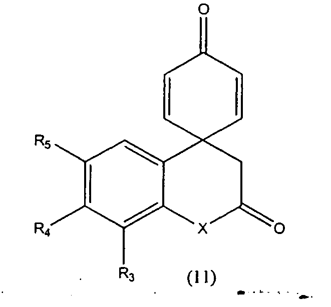 which is reacted with an amine of formula NHR6 where R6 is as defined in Claim 1 to give, by opening of the lactone, the corresponding amide of formula (12) 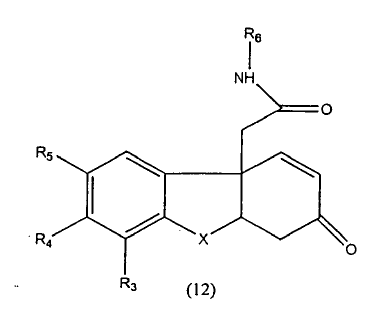 which is cyclized to give a compound of formula (1a), 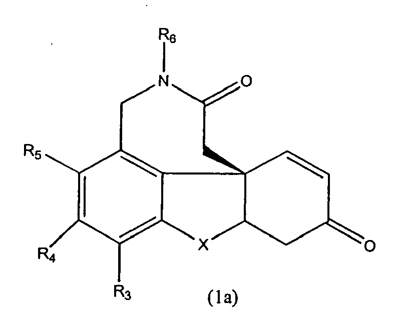 which is optionally subjected to a diastereoselective reduction to give the corresponding derivative of formula (1b), 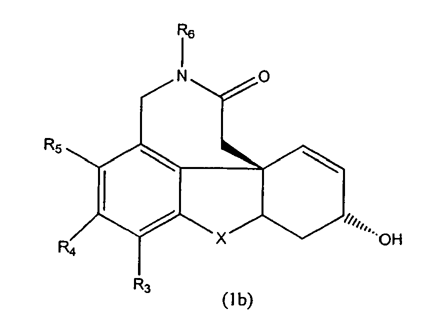 whose amide function can also be reduced, if desired, to give a compound of formula (1c)

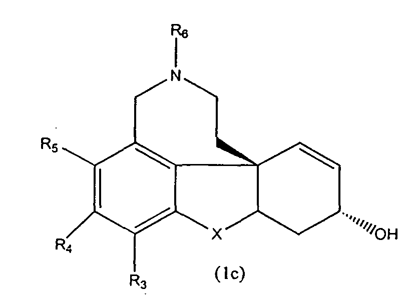

6. Method according to Claim 5, **characterized in that** the compound of formula (12) is resolved and then the synthesis is continued to give the compounds of formula (1a) to (1c) in their optically active forms.

7. Method according to Claim 5, **characterized in that** the compound of formula (1a) is resolved and then the synthesis is continued to give the compounds of formula (1b) to (1c) in their optically active forms.

8. Method according to any one of Claims 1 to 7, **characterized in that** galanthamine is prepared in the form of the racemate or of its optically pure isomers.

9. Compounds of formulae (11) and (12) 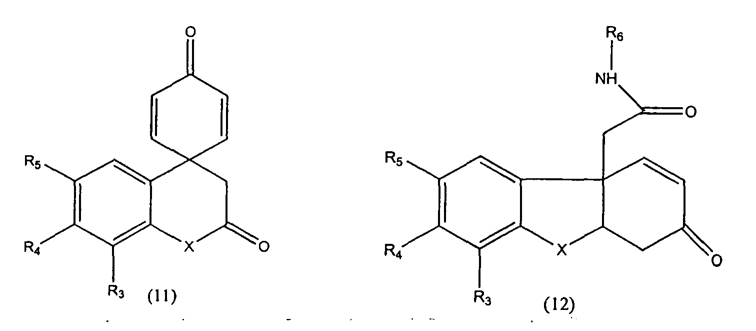 in which R₃, R₄, R₅, R₆, and X are as defined in Claim 1, useful as synthesis intermediates in a method according to any one of Claims 1 to 7.

10. Compounds according to Claim 9, **characterized in that** R₃=OCH₃, R₄=R₅=H and X=O, NH or N-CH₃.

11. Compounds of formulae (8) and (9) 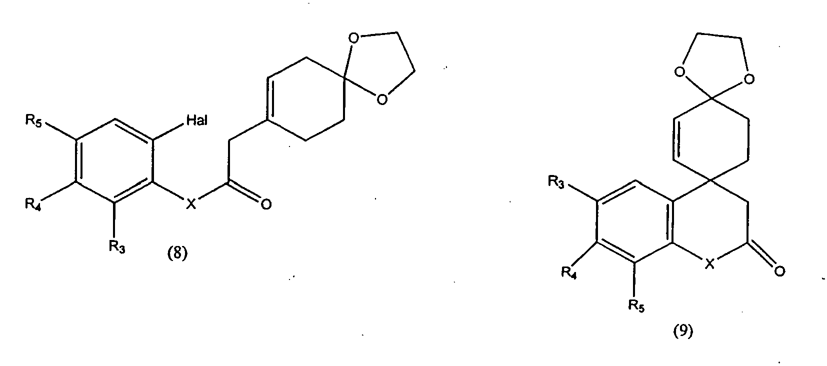 in which Hal, R₃, R₄, and R₅ are as defined in Claim 1 and X represents alternatively a sulfur atom or a nitrogen atom or an -SO group or an -SO₂ group or a group -NR₆ where R₆ is as defined in Claim 1 or represents an amine-protective group.

12. Compounds according to Claim 11, **characterized in that** Hal=I , R₃=OCH₃, R₄=R₅=H and X=NH or N-CH₃ .

13. Compounds selected from

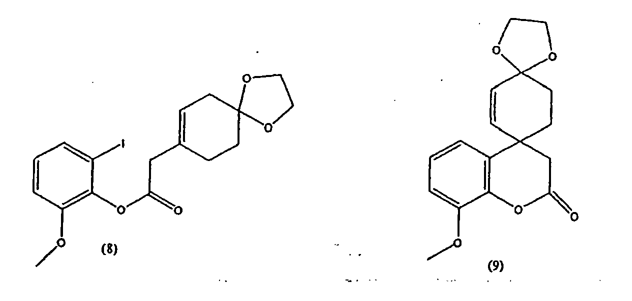

## Patentansprüche

1. Verfahren zur Synthese von Verbindungen der Formel (1), 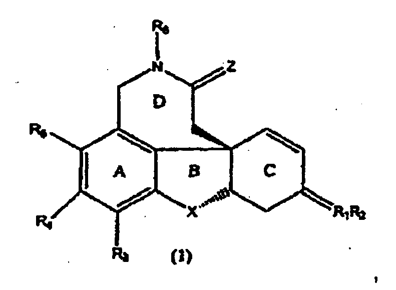 in welcher
entweder R₁ für ein Wasserstoffatom steht und R₂ für eine Hydroxygruppe steht oder R₁ und R₂ zusammen =O bilden,
R₃, R₄ und R₅ jeweils unabhängig voneinander für ein Wasserstoffatom, eine Hydroxygruppe oder eine (C₁-C₁₂)-Alkoxygruppe stehen,
R₆ für ein Wasserstoffatom, eine (C₁-C₁₂)-Alkylgruppe, eine Gruppe -(CH₂)ₙNR₇R₈ oder eine Gruppe -(CH₂)ₙN⁺R₇R₈R₉, worin n = 1 bis 12, R₇ und R₈ jeweils unabhängig voneinander für ein Wasserstoffatom, einen Cyanrest, (C₁-C₄)-Alkyl, (C₁-C₄)-Alkylcarbonyl stehen und R₉ für ein Wasserstoffatom, einen Cyanrest, (C₁-C₄)-Alkyl steht, steht,
Z entweder für zwei Wasserstoffatome oder ein Sauerstoffatom steht und
X entweder für ein Sauerstoffatom oder ein Schwefelatom oder ein Stickstoffatom oder eine Gruppe -SO oder eine Gruppe -SO₂ oder eine Gruppe -NR₆, worin R₆ wie zuvor definiert ist, steht oder für eine Amin-Schutzgruppe steht,
**dadurch gekennzeichnet, dass** es einen Schritt umfasst, in welchem man die Oxidation eines α,β-ethylenischen Ketons der Formel (10) 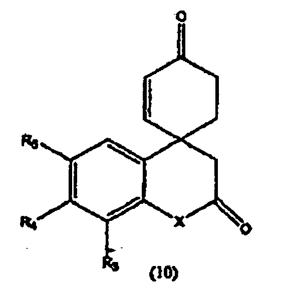 zu einem Spirodienon der Formel (11) 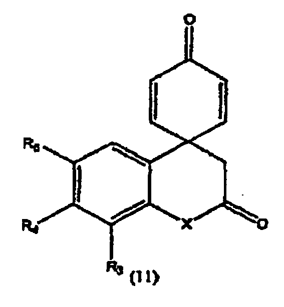 ausführt.

2. Verfahren nach Anspruch 1, **dadurch gekennzeichnet, dass** die Oxidation in Gegenwart von Phenylselensäureanhydrid, welches mit einem vorzugsweise anorganischen Träger gemischt ist, ausgeführt wird.

3. Verfahren nach einem der Ansprüche 1 und 2, **dadurch gekennzeichnet, dass** der Träger ausgewählt wird in der die Molekularsiebe und die Mischungen von Siliciumdioxid und Aluminiumoxid umfassenden Gruppe.

4. Verfahren nach Anspruch 3, **dadurch gekennzeichnet, dass** die Mischung von Siliciumdioxid und Aluminiumoxid eine 50/50-Mischung ist.

5. Verfahren nach einem der Ansprüche 1 bis 4, **dadurch gekennzeichnet, dass** man ein Derivat der Formel (6) 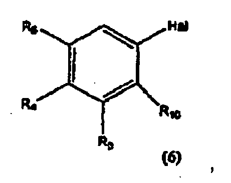 in welcher Hal für ein unter den Brom- und Iodatomen ausgewähltes Halogenatom steht, R₃, R₄ und R₅ wie in Anspruch 1 definiert sind und R₁₀ für eine Amingruppe oder eine Hydroxygruppe steht, mit (1,4-Dioxaspiro[4.5]dec-7-en-8-yl)-essigsäure der Formel (7) 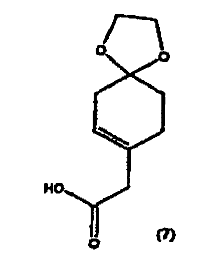 reagieren lässt und man eine Verbindung der Formel (8) 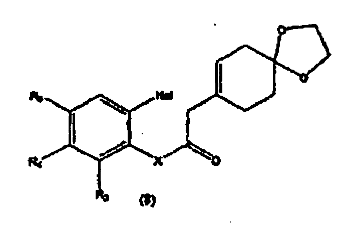 erhält, die man durch eine intramolekulare Heck-Reaktion cyclisiert, um eine Verbindung der Formel (9) 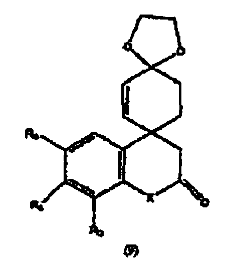 zu erhalten, in Gegenwart eines Katalysators mit Palladium oder einer Vorstufe von Palladium 0 und von zweizähnigen Alkylphosphin-Liganden in einem Lösemittel, man dann die Schutzgruppe von der Dioxolanfunktion der Verbindung der Formel (9) abspaltet, um das α,β-ethylenische Keton der Formel (10) 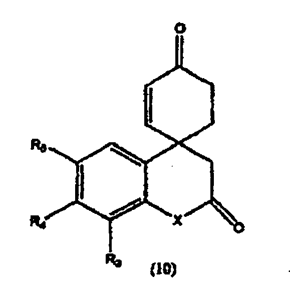 zu erhalten, das man in Gegenwart von Phenylselensäureanhydrid, zugesetzt zu einer 50/50-Mischung von Siliciumdioxid und Aluminiumoxid, oxidiert, und man eine Verbindung der Formel (11) erhält 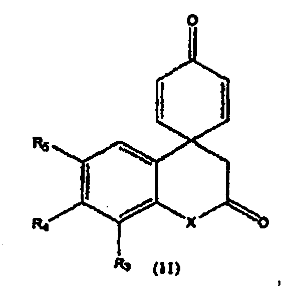 mit der man ein Amin der Formel NHR₆, worin Re wie in Anspruch 1 definiert ist, reagieren lässt, und man durch Öffnung des Lactons das entsprechende Amid der Formel (12) erhält 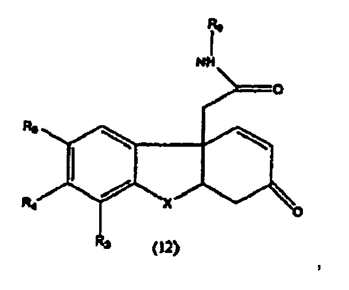 das man cyclisiert, um eine Verbindung der Formel (1a) 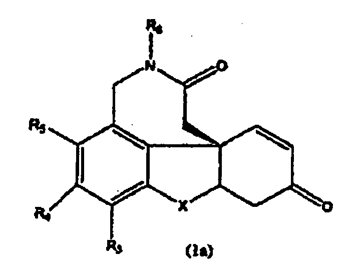 zu erhalten, die man gegebenenfalls einer diastereoselektiven Reduktion unterzieht, um das entsprechende Derivat der Formel (1b) 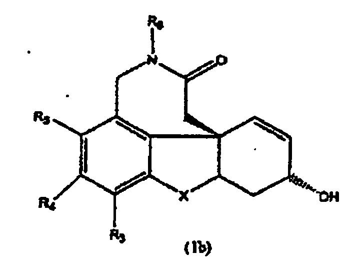 zu erhalten, von welchem man gleichfalls die Amidfunktion reduzieren kann, wenn man dies wünscht, um eine Verbindung der Formel (1c) 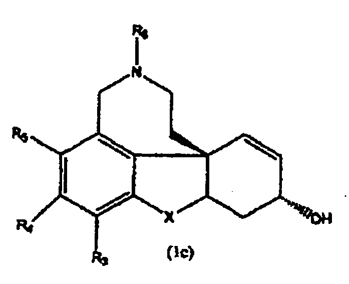 zu erhalten.

6. Verfahren nach Anspruch 5, **dadurch gekennzeichnet, dass** man die Enantiomerentrennung der Verbindung der Formel (12) ausführt, man dann die Synthese fortsetzt und man die Verbindungen der Formel (1 a) bis (1 c) in deren optisch aktiven Formen erhält.

7. Verfahren nach Anspruch 5, **dadurch gekennzeichnet, dass** man die Enantiomerentrennung der Verbindung der Formel (1a) ausführt, man dann die Synthese fortsetzt und man die Verbindungen der Formel (1b) bis (1c) in deren optisch aktiven Formen erhält.

8. Verfahren nach einem der Ansprüche 1 bis 7, **dadurch gekennzeichnet, dass** man das Galanthamin in Form eines Racemats oder von dessen optisch reinen Isomeren herstellt.

9. Verbindungen der Formeln (11) und (12) 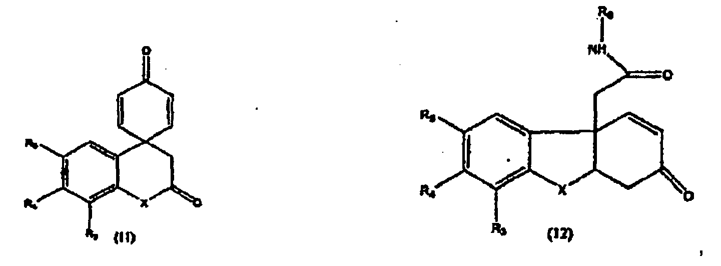 in welchen R₃, R₄, R₅, R₆ und X wie in Anspruch 1 definiert sind, die als Synthese-Zwischenstufen in einem Verfahren nach einem der Ansprüche 1 bis 7 nützlich sind.

10. Verbindungen nach Anspruch 9, **dadurch gekennzeichnet, dass** R₃ = OCH₃, R₄ = R₅ = H und X = O NH oder N-CH₃.

11. Verbindungen der Formeln (8) und (9) 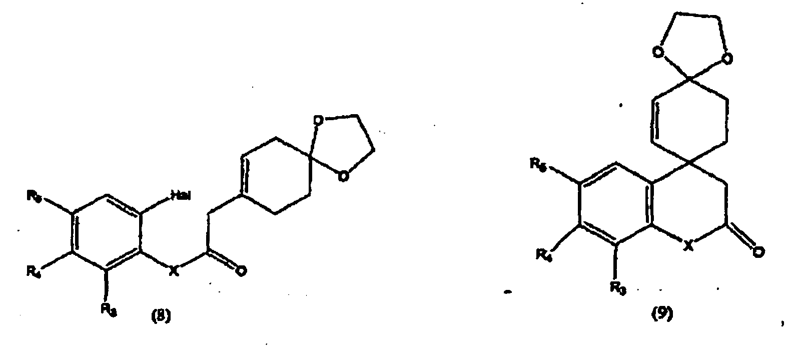 in welchen Hal, R₃, R₄ und R₅ wie in Anspruch 1 definiert sind und X entweder für ein Schwefelatom oder ein Stickstoffatom oder eine Gruppe -SO oder eine Gruppe -SO₂ oder eine Gruppe -NR₆, worin R₆ wie in Anspruch 1 definiert ist oder für eine Amin-Schutzgruppe steht, steht.

12. Verbindungen nach Anspruch 11, **dadurch gekennzeichnet, dass** Hal = I, R₃ = OCH₃, R₄ = R₅ = H und X = NH oder N-CH₃.

13. Verbindungen der Formeln (8) und (9)
